# EUROPEAN PATENT APPLICATION

(11) **EP 1 291 651 A1**
(43) Date of publication of application: **12.03.2003**
(21) Application number: 01941037.2
(22) Date of filing: 13.06.2001
(51) Int. Cl.: G01N 27/64, H01J 49/10, H01J 49/40

(54) **DEVICE FOR DETECTING CHEMICAL SUBSTANCE AND METHOD FOR MEASURING CONCENTRATION OF CHEMICAL SUBSTANCE**

(30) Priority: 14.06.2000 JP 2000178985
(71) Applicant: Mitsubishi Heavy Industries, Ltd., Tokyo 100-8315 (JP)
(72) Inventor: DANNO, Minoru, Advanced Tech. Res. Ce., Mitsubishi, Yokohama-shi, Kanagagawa 236-8515 (JP); YAMAKOSHI, Hideo Advanced Tech. Res. C. Mitsubishi, Yokohama-shi, Kanagagawa 236-8515 (JP); KOSHI, Mitsuo, Bunkyo-ku, Tokyo 113-0033 (JP)
(74) Representative: HOFFMANN - EITLE
(86) International application number: JP0105028
(87) International publication number: WO01096852

(57) **Abstract**

A vacuum ultraviolet ray generating unit (5) which generates vacuum ultraviolet rays used for ionizing a sample gas Gs in an ionization chamber (1) and a mass spectrometry unit (6) which accelerates the sample gas Gsi ionized by vacuum ultraviolet rays so as to measure the flight time of the accelerated substance.

## Description

### TECHNICAL FIELD

The present invention relates to a detector of chemical substances and a concentration-measuring method of chemical substances, and, more particularly, concerns a detector of chemical substances for detecting trace molecules such as halogenated organic compounds with high precision and a concentration-measuring method using such a detector.

### BACKGROUND ART

Noxious chemical substances even in a trace amount, such as chlorobenzenes and dioxins have been discharged in a state contained in exhaust gases discharged from incinerators and metal refining furnaces. There have been strong demands for methods detecting such trace noxious substances and for measuring the concentration thereof.

With respect to devices for detecting the above-mentioned chemical substances and for measuring the concentration thereof, conventional techniques such as a gas-chromatograph method and a mass spectrometry have been known, and of these, the mass spectrometry is superior in its shorter measuring time in comparison with the gas-chromatograph method.

In the mass spectrometry, a sample gas is ionized by using a plasma derived from an RF discharge (high frequency discharge) or an electron beam from an electron gun, and the ions are instantaneously accelerated to be subjected to mass separation so that the substance is identified by measuring the flight time corresponding to its mass number.

In the above-mentioned flight-time measuring type spectrometry, during the ionizing process of the sample gas, substances other than the substance to be detected are ionized, the mass of the substance to be detected or a substance not to be detected is decomposed into a molecular or an atom that is smaller than the original substance, resulting in complex fragments that are decomposed and generated; thus, it becomes difficult to identify a specific substance and there is reduction in the measuring sensitivity.

For this reason, techniques for preventing substances other than the detection subject substance in a sample gas from being ionized have been developed. Moreover, a resonance multi-photon ionization method has been known in which-a laser light beam which is adjusted to the light absorbing wavelength of the substance to be measured is applied, and the substance is selectively subjected to multi-photoionization.

However, in the resonance multi-photon ionization method that can improve the measuring sensitivity, among chlorobenzenes and dioxins, with respect to those substances having much chloride such as dichlorobenzene and trichlorobenzene, there is degradation in the efficiency of ionization and the resulting reduction in the measuring sensitivity; therefore, in order to compensate for the degradation in ionization, an ultrashort pulse laser is required. For this reason, in order to measure specific substances as described above, the conventional devices tend to become expensive.

Therefore, the present invention has been achieved in order to solve the above problems, and its objects are to improve the ionization efficiency to a specific substance, to prevent other substances having ionizing energy higher than photon energy from being ionized, to improve the measuring sensitivity by regulating the generation of fragments of the specific substance, and to provide a detector of chemical compounds and a concentration-measuring method of chemical compounds which can reduce the device costs and simplify the device.

### DISCLOSURE OF THE INVENTION

A detector of chemical compounds according to the present invention, which has a premise that, to a specific substance having ionizing energy, vacuum ultraviolet rays having photon energy that exceeds the ionizing energy is applied so that the substance is ionized by one photon energy, is provided with a vacuum ultraviolet ray generating unit which generates vacuumultraviolet rays for ionizing a sample gas, and a mass spectrometry unit which measures the flight time of a substance that has been accelerated by accelerating the sample gas ionized by the vacuum ultraviolet rays. The unit which finds the mass of the accelerated substance based upon the flight time and identifies the substance is prepared as a known unit.

The vacuum ultraviolet ray generation unit may be constituted by a light-emitting lamp using a gas discharge, and is allowed to select a substance to be ionized by changing the quantity of photon energy to be generated by changing the kind of a gas to be discharged. Moreover, the vacuum ultraviolet ray generating unit may generate a laser beam or its higher harmonics. In these cases also, the quantity of photon energy to be generated is varied by a variable laser so as to select a substance to be ionized.

It is particularly effective to add to the detector of chemical substances of the present invention an ion trap for generating a high frequency electric field therein. In this case, the sample gas accumulated in the ion trap is accelerated so that specific molecules that have been selectively ionized by the ion trap are condensed. This condensation makes it possible to increase the probability of existence of the specific ions.

The substances to be detected are halogenated organic compounds such as chlorobenzenes and dioxins, and the ionizing energy of these is in the range of 9 to 10 eV, and the photon energy of vacuum ultraviolet rays to be applied is preferably set to approximately 10 eV so as to provide energy not less than the ionizing energy and also to suppress fragments resulting from decompositions at the time of ionization of other mixed substances and ionization of the substance to be detected. Moreover, as has been described earlier, it is further preferable to add a process for condensing the detection subject substance that has been subjected to application of vacuum ultraviolet rays in a high frequency electric field.

### BRIEF DESCRIPTION OF THE DRAWINGS

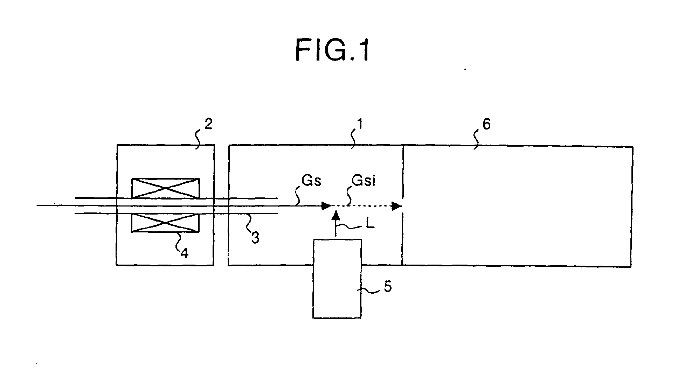

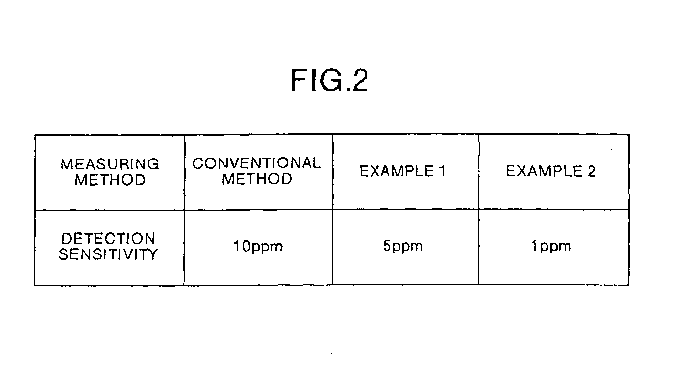

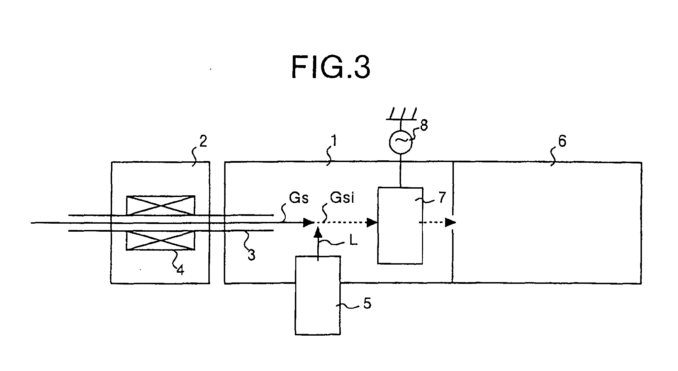

Fig. 1 is a block diagram that shows one preferred embodiment of a detector of chemical substances according to the present invention; Fig. 2 is a graph that shows the results of experiments carried out on the detector of chemical substances; and Fig. 3 is a block diagram that shows another preferred embodiment of a detector of chemical substances according to the present invention.

### BEST MODES FOR CARRYING OUT THE INVENTION

Referring to attached drawings, an explanation will be given of preferred embodiments of the present invention in detail.

Fig. 1 shows one preferred embodiment of a detector of chemical substances according to the present invention.

The detector of chemical substances of the present invention is provided with an ionization chamber 1 together with a gas injection device 2. The gas injection device 2 has a gas discharging pipe 3. The gas discharging pipe 3 is formed by an open-close valve using an orifice such as a pulse valve or a capillary pipe. A sample gas Gs, directed to the gas discharging pipe 3 from the injection side, is further directed to the ionization chamber 1.

It is assumed that the sample gas Gs contains halogenated organic compounds (hereinafter, referred to detection subject substances) . Here, "halogenated organic substances" refer to organic chemical compounds having at least one halogen (chlorine, bromine, iodine) within a molecule, and examples thereof include: halogenated benzenes (monochlorobenzene, dichlorobenzene, trichlorobenzene, tetrachlorobenzene, pentachlorobenzene, hexachlorobenzene, etc.), halogenated phenols (chlorophenol, etc.), halogenated hydrocarbon compounds (tetrachloroethylene, etc.), halogenated naphthalates (chloronaphthalene, etc.), halogenated biphenyls (chlorobiphenyl, etc.), dioxins (polychlorodibenzoparadioxin, polychlorodibenzofuran, etc), etc. A heater 4 is placed on the periphery of the gas discharging pipe 3. The heater 4 is a heating device for preventing the detection subject substances from adhering to the inner wall of the gas discharging pipe 3.

The ionization chamber 1 is provided with a vacuum ultraviolet ray lamp 5 serving as a vacuum ultraviolet ray generating unit. The vacuum ultraviolet ray lamp 5 generates vacuum ultraviolet rays L by a discharge of a rare gas such as Ar, Kr and Xe, and a gas obtained by applying H₂, O₂, Cl₂ or the like to Ar or He.

By changing the kind of a gas for discharge in response to the ionizing energy of a substance to be detected, the vacuum ultraviolet ray lamp 5 changes the quantity of photon energy of the generated vacuum ultraviolet rays so that it is possible to prevent the ionization of a mixed substance having ionizing energy higher than the quantity of photon energy and also to suppress fragments of the detection subject substance. Moreover, instead of the vacuum ultraviolet ray lamp 5, a laser or its high harmonics may be used. In this case, by changing the quantity of photon energy to be generated by using a wavelength variable laser, it becomes possible to select the substance to be ionized. Conventionally known lasers may be used as the wavelength variable laser.

The sample gas Gs, directed to the ionization chamber 1, is subjected to the application of the vacuum ultraviolet rays L outputted by the vacuum ultraviolet ray lamp 5, and ionized by receiving photon energy from the vacuum ultraviolet rays L.

The sample gas Gsi thus ionized is directed into a mass spectrometry device 6 placed next to the ionization chamber 1. Upon receipt of an instantaneous accelerating voltage, the ionized substance in the sample gas Gsi is directed to the mass spectrometry device 6, and allowed to fly inside the mass spectrometry device 6. The mass spectrometry device 6 measures its flight time. The flight time and the mass of the flying substance have the corresponding relationship in height so that the mass of the flying substance is detected by the flight time and the substance is identified by the mass.

### Example 1

Monochlorobenzene diluted by helium gas is adjusted in its concentration, and used as a sample gas Gs. The sample gas Gs to be directed to the gas discharging pipe 3 is heated by the heater 4, and then directed to the ionization chamber 1.

The sample gas Gs, directed to the ionization chamber 1, is ionized upon receipt of application of the vacuum ultraviolet rays L by the vacuum ultraviolet ray lamp 5. The vacuum ultraviolet ray lamp 5 discharges a mixed gas of H₂ and Ar in the form of microwaves as vacuum ultraviolet rays L, to generate light having photon energy in a level of 10.2 eV.

The sample gas Gsi, ionized upon receipt of such vacuum ultraviolet rays L, is subjected to a spectrometry process by the spectrometry device 6. The minimum detection concentration of monochlorobenzene, obtained as the results of spectrometry, was 5 ppm, as shown in Fig. 2. The same figure also shows the results of spectrometry in the case of the ionization using an electron beam of 70 eV that is a conventional method, and the minimum detection concentration is 10 ppm.

Fig. 3 shows another embodiment of a detector of chemical substances according to the present invention, and the arrangement of a mass spectrometry device 6 including an ionization chamber 1, a gas injection device 2, a gas discharging pipe 3, a heater 4 and a vacuum ultraviolet ray lamp 5 is the same as that shown in Fig. 1.

In the embodiment shown in Fig. 3, an ion trap 7 in which a high frequency electric field is formed is further added thereto. The ion trap 7 is a conventional technique, and a high frequency voltage is applied to the ion trap 7 by a high frequency power supply 8. The frequency and voltage of the high frequency power supply 8 and the mass number of ions to be condensed and accumulated in the ion trap 7 have the corresponding relationship. Substances other than a specific substance to be accumulated by receiving a voltage of a specific frequency are ejected from the ion trap 7, while the specific substance is condensed in an electric field with a high frequency voltage.

After the condensed specific substance has been accumulated for a predetermined time (several seconds), an accelerating voltage is applied to the specific substance so that the condensed specific ionized substance with a high concentration is directed to the mass spectrometry device 6 and allowed to fly in the mass spectrometry device 6 so that the flight time is measured.

The flight time and the mass of the flying substance have the corresponding relationship in height. These mass spectrometry device and mass spectrometry method are known units referred to as the flight-time measuring type mass spectrometry device and the flight-time measuring type mass spectrometry method.

### Example 2

Sample gas Gs, directed to the ionization chamber 1, is ionized upon receipt of application of the vacuum ultraviolet rays L from the vacuum ultraviolet ray lamp 5 in the same manner as Example 1. Here, the time during which the ionized monochlorobenzene that is a specific substance has been accumulated while receiving an applied voltage with a specific frequency inside the ion trap 7 is two seconds. As shown in Fig. 2, the minimum detection concentration as the results of analysis in the flight-time measuring type mass spectrometry device 6 was 1 ppm.

As is understood by the above-mentioned explanation, according to the detector of chemical substances and the concentration measuring method of chemical substances, the ionization efficiency of a specific substance such as a halogenated organic compound is improved so that it is possible to prevent the ionization of other substances having ionizing energy higher than photon energy and also to suppress the generation of fragments of the specific substance; thus, it becomes possible to improve the measuring sensitivity and also to reduce the costs of the device and simplify the device.

Moreover, the application of an ion trap makes it possible to increase the detection sensitivity and to minimize the minimum detection density and consequently to carry out a concentration detecting process with high precision.

### INDUSTRIAL APPLICABILITY

As described above, the detector of chemical compounds and the concentration measuring method of chemical substances of the present invention are suitable for detecting trace molecules such as a halogenated organic compound with high precision.

## Claims

1. A detector of chemical compounds comprising:
a vacuum ultraviolet ray generating unit which generates vacuum ultraviolet rays for ionizing a sample gas in an ionizing chamber; and
a mass spectrometry unit which measures the flight time of a substance that has been accelerated by accelerating the sample gas ionized by the vacuum ultraviolet rays.

2. The detector of chemical compounds according to claim 1, wherein the vacuum ultraviolet ray generating unit is a light-emitting lamp using a gas discharge.

3. The detector of chemical compounds according to claim 2, wherein the vacuum ultraviolet ray generating unit is allowed to select a substance to be ionized by changing the quantity of photon energy to be generated by changing the kind of a gas to be discharged.

4. The detector of chemical compounds according to claim 1, wherein the vacuum ultraviolet ray generating unit generates a laser beam or its higher harmonics.

5. The detector of chemical compounds according to claim 2, wherein the vacuum ultraviolet ray generating unit varies the quantity of photon energy to be generated by using a variable laser so as to select a substance to be ionized.

6. The detector of chemical compounds according to any one of claims 1 to 5, wherein the chemical substance to be detected is a halogenated organic compound and the halogenated organic compound is ionized by vacuum ultraviolet rays having photon energy of not less than the ionizing energy thereof.

7. The detector of chemical compounds according to any one of claims 1 to 5, comprising an ion trap for generating a high frequency electric field therein so that the sample gas accumulated in the ion trap is accelerated.

8. A concentration-measuring method of chemical substances, wherein to a specific substance having ionizing energy, vacuum ultraviolet rays having photon energy exceeding the energy are applied so that the substance is ionized by one photon energy, the ionized sample gas is accelerated, and based upon the measurement of flight time of the accelerated substance, the substance is identified and the concentration is measured.

9. A concentration-measuring method of chemical substances, wherein to a specific substance having ionizing energy, vacuum ultraviolet rays having photon energy exceeding the energy are applied so that the substance is ionized by one photon energy, the ionized sample gas is condensed by an ion trap, the ionized gas thus concentrated is accelerated, and based upon the measurement of flight time of the accelerated substance, the substance is identified and the concentration is measured.
